# EUROPEAN PATENT APPLICATION

(11) **EP 3 561 497 A1**
(43) Date of publication of application: **30.10.2019**
(21) Application number: 17883903.1
(22) Date of filing: 31.10.2017
(51) Int. Cl.: G01N 27/12, G01N 27/416

(54) **GAS SENSOR**

(30) Priority: 20.12.2016 JP 2016246257
(71) Applicant: NGK Spark Plug Co., Ltd., Nagoya-shi, Aichi 467-8525 (JP)
(72) Inventor: INOUE Tsuyoshi, Nagoya-shi Aichi 467-8525 (JP); UEKI Masatoshi, Nagoya-shi Aichi 467-8525 (JP); SHICHIDA Takafumi, Nagoya-shi Aichi 467-8525 (JP); NISHIO Kenji, Nagoya-shi Aichi 467-8525 (JP); AOYAMA Shigeya, Nagoya-shi Aichi 467-8525 (JP)
(74) Representative: J A Kemp LLP
(86) International application number: PCT/JP2017/039262
(87) International publication number: WO 2018/116641

(57) **Abstract**

Disclosed is a gas sensor capable of promoting gas replacement around a sensor element within a casing and attaining improved detection response. The gas sensor 1 includes: a wiring board 50; a sensor element 24 mounted on the wiring board and electrically connected to the wiring board by a plurality of conductive members 28; a casing 22 installing the sensor element and formed with inlet and outlet ports 22a and 22b; and a pretreatment unit 10 configured to adjust the concentration of a specific gas component in a measurement gas G and feed the measurement gas to the inlet port 22a. The inlet and outlet ports are located at positions outward and upward of the sensor element. A protrusion 22p is provided protruding toward the inside of the casing so as to narrow a flow path from the inlet port to the outlet port. A height h1 from the sensor element to a distal end of the protrusion is lower than heights h2 and h3 from the sensor element to the inlet and outlet ports. The protrusion is disposed more inside than respective top portions 28p of the plurality of conductive members, which are located upward of the sensor element, without being in contact with the conductive members.

## Description

### Field of the Invention

The present invention relates to a gas sensor for detecting the concentration of a specific gas component in a measurement gas.

### Background Art

There is conventionally known a gas sensor for detecting the concentration of a specific gas component in a measurement gas (see Patent Document 1). This gas sensor is configured to supply a predetermined amount of air as the measurement gas into a chamber, after performing pretreatment on the measurement gas in the chamber for combustion and removal of combustible gas such as CO, bring the measurement gas into contact with a sensor element and then detect the concentration of NOx in the measurement gas.

### Prior Art Documents

### Patent Document

Patent Document 1: Japanese Laid-Open Patent Publication No. H10-300702 (FIG. 2)

### Summary of the Invention

### Problems to be Solved by the Invention

In Patent Document 1, the chamber is provided with air inlet and outlet ports; and the sensor element is arranged in the chamber. When the flow rate of the measurement gas in the chamber becomes slow, the detection response of the gas sensor may be lowered due to insufficient gas replacement around the sensor element.

In view of the foregoing, it is an object of the present invention to provide a gas sensor capable of promoting gas replacement around a sensor element within a casing and thereby attaining improved response in detection of a specific gas component.

### Means for Solving the Problems

To achieve the above object, the present invention provides a gas sensor comprising: a wiring board extending in a longitudinal direction; a sensor element configured to detect a specific gas component in a measurement gas, the sensor element being disposed inside an outer circumference of one surface of the wiring board and being electrically connected to the wiring board by a plurality of conductive members; a casing defining an installation space in which the sensor element is installed, the casing having formed thereon an inlet port through which the measurement gas is introduced into the installation space and an outlet port through which the measurement gas is discharged out from the installation space; and a pretreatment unit configured to pretreat the measurement gas so as to adjust the concentration of the specific gas component in the measurement gas, and then, feed the pretreated measurement gas to the inlet port,
wherein, assuming that a direction in which the sensor element faces the wiring board is a downward direction, the inlet and outlet ports are located at positions outside an outer circumference of the sensor element and upward of the sensor element,
wherein the gas sensor comprises a protrusion provided at a position midway in a flow path of the installation space from the inlet port to the outlet port and facing the sensor element such that the protrusion protrudes toward the inside of the casing so as to narrow the flow path,
wherein a height from the sensor element to a distal end of the protrusion in a direction perpendicular to the longitudinal direction is lower than heights from the sensor element to the inlet and outlet ports in the direction perpendicular to the longitudinal direction, and
wherein the protrusion is disposed more inside than respective top portions of the plurality of conductive members, which are located upward of the sensor element, without being in contact with the conductive members.

In the above gas sensor, the protrusion is provided to narrow the flow path of the installation space from the inlet port to the outlet port. Thus, the flow rate of the measurement gas in a part of the flow path facing the sensor element is increased by the Venturi effect so as to promote gas replacement around the sensor element and thereby attain improved response in detection of the specific gas component. As the protrusion is arranged in non-contact with the conductive members 28, a defective condition such as a break in the conductive member is prevented from being caused by contact of the protrusion with the conductive member. In the presence of a clearance between the protrusion and the conductive members, there is prevented interference with the flow of the measurement gas in the vicinity of the protrusion.

In the present invention, the gas sensor may be provided wherein the casing is formed from a metal plate; and wherein the protrusion is formed integral with the casing. In this case, the gas sensor is improved in productivity and reduced in part count as compared to the case where the protrusion is formed as a separate piece and attached to the casing, while the casing is provided with good heat resistance. In the case of press-forming etc., the protrusion is easily formed in a smooth shape with rounded-off corners so as to further prevent interference with the flow of the measurement gas in the vicinity of the protrusion.

### Effects of the Invention

The gas sensor according to the present invention promotes gas replacement in the vicinity of the sensor element within the casing and attains improved response in detection of the specific gas component.

### Brief Description of Drawings

FIG. 1 is a block diagram showing the overall structure of a gas sensor according to an embodiment of the present invention.
FIG. 2 is an exploded perspective view of the gas sensor according to the embodiment of the present invention.
FIG. 3 is a cross-sectional view taken along line A-A of FIG. 2.
FIG. 4 is an exploded perspective view of a gas sensor according to one modification of the embodiment of the present invention.
FIG. 5 is an exploded perspective view of a gas sensor according to another modification of the embodiment of the present invention.

### Description of Embodiments

Hereinafter, the present invention will be described in detail below with reference to the drawings.

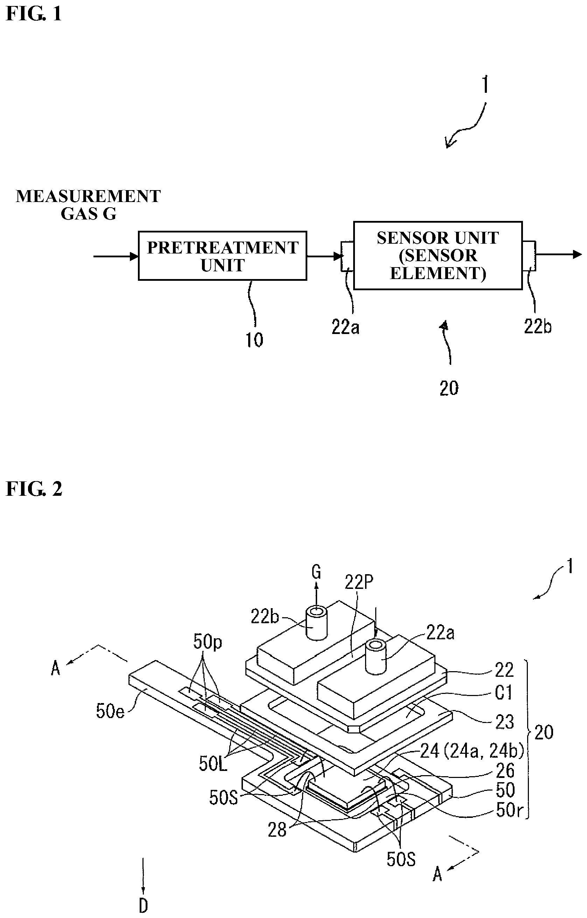

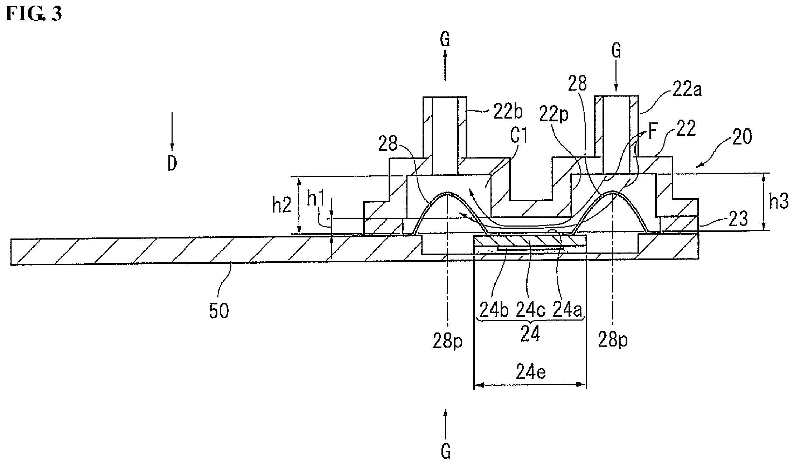

FIG. 1 is a block diagram showing the overall structure of a gas sensor 1 according to an embodiment of the present invention. FIG. 2 is an exploded perspective view of the gas sensor 1. FIG. 3 is a cross-sectional view taken along line A-A of FIG. 2.

As shown in FIG. 1, the gas sensor 1 includes a pretreatment unit 10, a sensor unit 20 and a gas flow pipe (not shown) connecting the pretreatment unit 10 and the sensor unit 20. A measurement gas G (such as human breath) is first introduced into the pretreatment unit 10. The pretreatment unit 10 performs pretreatment to adjust the concentration of a specific gas component (such as NO₂) in the measurement gas G, and then, feeds the pretreated measurement gas to an inlet port 22a of the sensor unit 20. The pretreatment unit 10 can be of known configuration having a catalyst capable of adjusting the concentration of the specific gas component in the measurement gas. A detailed explanation of the pretreatment unit of the present embodiment will be hence omitted herefrom. On the other hand, the sensor unit 20 detects the specific gas component (more specifically, the concentration of the specific gas component) in the pretreated measurement gas and then discharges the measurement gas out from an outlet port 22b.

The configuration of the sensor unit 20 will be next explained in detail below with reference to FIGS. 2 and 3. In FIGS. 2 and 3, a direction in which a sensor element 24 faces a wiring board 50 is defined as a downward direction D.

As shown in FIG. 2, the sensor unit 20 includes: a casing 22 formed of a metal material in a substantially rectangular box shape with an opening at a bottom surface thereof facing the downward direction D; a seal member (such as packing) formed in a rectangular frame shape and bonded to a flange portion of the casing 22; a sensor element 24 installed in the casing 22; an adhesive layer 26; and a ceramic wiring board 50. By bonding and fixing the flange portion of the casing 22 and an outer circumferential portion of a top surface of the wiring board 50 to a frame body of the seal member 23 via an adhesive (not shown), the opening of the casing 22 is closed with the wiring board 50 so that the inner space of the casing 22 is defined as an installation space (chamber) C1. The inlet port 22a and the outlet port 22b are each formed protrudingly in a pipe shape on a top surface of the casing 22 and are spaced apart from each other. One ends of the inlet and outlet ports 22a and 22b are in communication with the installation space C1. The inlet port 22a allows introduction of the measurement gas G therethrough into the installation space C1, whereas the outlet port 22b allows discharge of the measurement gas therethrough out from the installation space C1. A portion of the top surface of the casing 22 between the inlet port 22a and the outlet port 22b is depressed by press forming into a substantially rectangular box shape. By such a depressed portion, there is formed a protrusion 22p inwardly protruding from an inner surface of the casing 22 (see FIG. 3).

The sensor element 24 is substantially rectangular plate-shaped. As shown in FIG. 3, the sensor element 24 has an integral structure in which a detection portion 24a and a heater 24b are respectively disposed and stacked on a top surface side (upward side of FIG. 2) and a bottom surface side of a base portion 24c. A recess 50r is formed in a center portion of the top surface of the wiring board 50. The sensor element 24 is mounted on the wiring board 50 such that the heater 24b side of the sensor element 24 abuts the recess 50r via the adhesive layer 26. In other words, the sensor element 24 is disposed inside the outer circumference of the top surface of the wiring board 50. Herein, the top surface of the wiring board 50 corresponds to the claimed one surface.

The wiring board 50 extends in a longitudinal direction (i.e. horizontal direction of FIG. 2). One end portion 50e of the wiring board 50 (on left side of FIG. 2) is formed, with a narrower width than the casing 20, so as to extend outwardly (to left side of FIG. 2). A plurality of electrode pads 50p are disposed on top and bottom surface sides of the end portion 50e. These electrode pads 50p are respectively electrically connected to wiring lines (lead conductors) 50L on the top and bottom surfaces of the wiring board 50. The wiring lines 50L are connected at one ends thereof to a plurality of element peripheral pads 50s around the recess 50r. As shown in FIG. 2, the wiring lines 50L on the bottom surface of the wiring board 50 are routed to the top surface side of the wiring board 50 at the other end portion of the wiring board 50 (opposite from the end portion 50e), and then, connected to the element peripheral pads 50s on the top surface of the wiring board 50. Output terminals of the detection portion 24a and conduction terminals of the heater 24b are respectively electrically connected to the element peripheral pads 50s of the wiring board 50 by conductive members (more specifically, wires 28). Namely, the output terminals of the detection portion 24a and the conduction terminals of the heater 24b are respectively wire-bonded to the corresponding element peripheral pads 50s of the wiring board 50. With this connection structure, an electrical signal outputted from the detection portion 24 is taken out to an external device through the wiring lines 50L and electrode pads 50p of the wiring board 50; and the heater 24b is energized and heated with the supply of power from an external power source through the electrode pads 50p and wiring lines 50L of the wiring board 50.

When the measurement gas G in which the concentration of the specific gas component has been adjusted by the pretreatment unit 10 is brought into contact with the detection portion 24a, the detection portion 24 detects the concentration of the specific gas component. As the electrical characteristic of the detection portion 24a changes according to the concentration of the specific gas component, the concentration of the specific gas component is determined by detection of the changing electrical signal. The heater 24b generates heat by energization thereof and thereby heats the detection portion 24a to a desired operating temperature. The base portion 24c is formed as an insulating wiring board. The detection portion 24a is formed of e.g. a metal oxide semiconductor material. The heater 24b is formed as e.g. a heating resistor of platinum etc. in a meandering pattern shape on the surface of the base portion 24c. The detection portion 24 may be of known mixed-potential type sensor configuration in which a pair of electrodes are disposed on a solid electrolyte body.

As shown in FIG. 3, the inlet and outlet ports 22a and 22b are located at positions outside the outer circumference 24e of the sensor element 24 and upward of the sensor element 24. The protrusion 22p is formed at a position midway in a flow path F of the installation space C1 from the inlet port 22a to the outlet port 22b and facing the sensor element 24 (i.e. inside the outer circumference 24e), and protrudes toward the inside of the casing 22 so as to narrow the flow path F. A height h1 from the sensor element 24 (more specifically, the detection portion 24a on the upward end of the sensor element 24) to a distal end of the protrusion 22p in a direction perpendicular to the longitudinal direction of the wiring board 50 is set lower than a height h2 from the sensor element 24 to the inlet port 22a in the direction perpendicular to the longitudinal direction of the wiring board 50 and a height 3 from the sensor element 24 to the outlet port 22b in the direction perpendicular to the longitudinal direction of the wiring board 50.

Further, the protrusion 22p is formed with the same width dimension (i.e. length from the front to the back of the paper of FIG. 3) as that of the installation space C1 in the present embodiment. In other words, the width dimension of the protrusion 22p is set equal to an inside dimension between two opposed side surfaces of the substantially rectangular box-shaped casing 22 defining the installation space C1. The protrusion 22p is hence located substantially perpendicular to the flow of the measurement gas G from the inlet port 22a to the outlet port 22b so that the measurement gas G is brought guided to the sensor element 24 by contact with the inlet port 22a side surface of the protrusion 22p. Thus, the flow rate of the measurement gas G in a part of the flow path F facing the sensor element 24 is increased by the Venturi effect so as to promote gas replacement around the sensor element 24 and thereby attain improved response in detection of the specific gas component.

Furthermore, the protrusion 22p is disposed more inside than respective top portions 28 of the plurality of conductive members 28, which are located upward of the sensor element 24, without being in contact with these conductive members 28. Consequently, a defective condition such as a break in the conductive member 28 is prevented from being caused by contact of the protrusion 22p with the conductive member 28. In the presence of a clearance between the protrusion 22p and the conductive member 28, there is prevented interference with the flow of the measurement gas G in the vicinity of the protrusion 22p. Each of the conductive members 28 extends from the element peripheral pad 50s to the outer circumference of the sensor element 24, which is located more inside than the element peripheral pad 50s, and has an upward-convex curved shape with the top portion 28p between the element peripheral pad 50s to the sensor element 24.

In the present embodiment, the casing 22 is formed from a metal plate; and the protrusion 22p is formed integral with the casing 22 by press forming of the metal plate. The gas sensor is accordingly improved in productivity and reduced in part count as compared to the case where the protrusion 22p is formed as a separate piece and attached to the casing 22, while the casing 22 is provided with good heat resistance. In the case where the protrusion 22p is formed by press-forming etc. as in the present embodiment, the protrusion 22p is easily formed in a smooth shape with rounded-off corners so as to further prevent interference with the flow of the measurement gas G in the vicinity of the protrusion 22p.

The present invention is not limited to the above-described embodiment and includes all changes, modifications and equivalents falling within the technical idea and scope of the present invention.

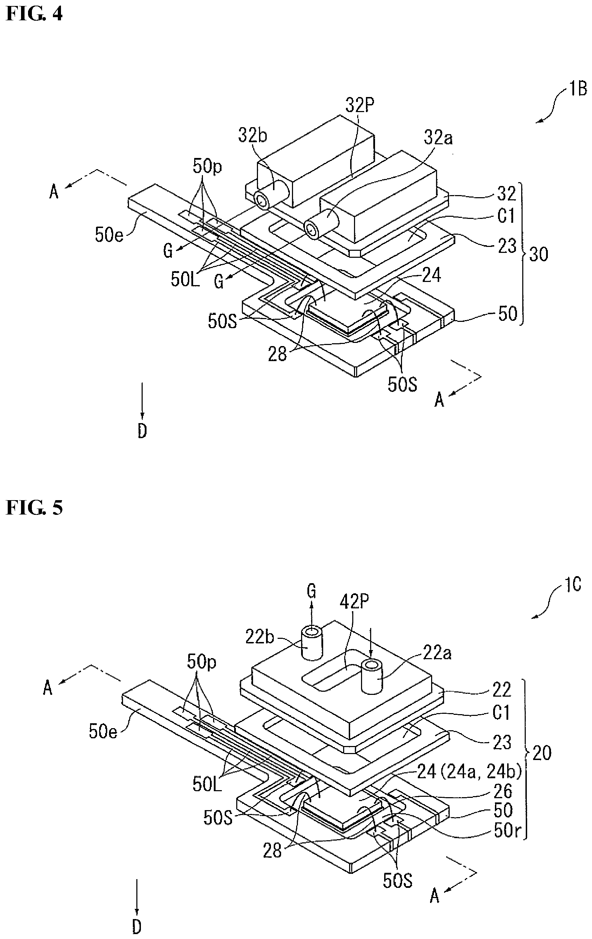

For example, a gas sensor 1B may be provided with a sensor unit 30 in which a casing 32 has inlet and outlet ports 32a and 32b protruding from a side surface thereof as shown in FIG. 4. The inlet and outlet ports 32a and 32b can be in any arrangement as long as the inlet and outlet ports 32a and 32b are located at positions outside the outer circumference 24e of the sensor element 24 and upward of the sensor element 24. The inlet and outlet ports 32a and 32b may alternatively be arranged on opposed side surfaces of the casing 32. In this modified embodiment, a protrusion 32p is formed at a position midway in the flow path F of the installation space C1 from the inlet port 32a to the outlet port 32b and facing the sensor element 24, and protrudes toward the inside of the casing 32 so as to narrow the flow path F. Further, the protrusion 32p is disposed more inside than the respective top portions of the plurality of conductive members 28, which are located upward of the sensor element 24, without being in contact with the conductive members 28 as in the case of the above embodiment. It is noted that, in FIG. 4, the same reference numerals are used to refer to the same parts and portions as those in the sensor unit 20 of FIG. 2.

Although the protrusion 22p is formed with the same width dimension as that of the installation space C1 in the gas sensor 1 of FIGS. 2 and 4, the width dimension of the protrusion can be set within the range that produces the effect of promoting gas replacement around the sensor element 24 and attaining improved response in detection of the specific gas component. As shown in FIG. 5, a gas sensor 1C may be provided another embodiment in which a protrusion 42 is formed on the casing 22, at a position facing the sensor element 24, with a smaller width dimension than that of the installation space C1. Even in this case, the protrusion 42p is disposed more inside than the respective top portions of the plurality of conductive members 28, which are located upward of the sensor element 24, without being in contact with the conductive members 28 as in the case of the above embodiment. It is noted that, in the gas sensor 1C of FIG. 5, the same reference numerals are used to refer to the same parts and portions as those in the gas sensor 1 of FIG. 2.

The shapes, materials and the kinds of the gas sensor and its constituent parts such as pretreatment unit, casing, sensor element, conductive members and protrusion are not limited to those of the above embodiment. The number of protrusions is not also particularly limited.

In the above embodiment, the flange portion of the casing 22 and the outer circumferential portion of the top surface of the wiring board 50 are bonded and fixed to the frame body of the seal member 23 via the adhesive. However, the bonding and fixing structure of the casing 22, the seal member 23 and the wiring board 50 is not limited to this type. For example, the gas sensor 1 may be constructed by applying a force (biasing force) externally of the casing 22 to the wiring board 50 with the use of another member (such as bolt and nut) and thereby fixing the casing 22, the seal member 23 and the wiring board 50 in position respectively relative to one another without using an adhesive.

Moreover, the protrusion 22p, 32p, 42p is formed integral with the metal casing 22, 32 by press forming in the above embodiment, but is not limited to this configuration. It is feasible, for example, to provide the casing with a flat top surface and join (more specifically, weld) a protruding strip piece to a predetermined position on the inner side of the top surface of the casing such that the protruding strip piece serves as the protrusion in the present invention.

### Description of Reference Numerals

- 1, 1B, 1C:: Gas sensor
- 10:: Pretreatment unit
- 22, 32:: Casing
- 22a, 32a:: Inlet port
- 22b, 32b:: Outlet port
- 22p, 32p, 42p:: Protrusion
- 24:: Sensor element
- 24e:: Outer circumference of sensor element
- 28:: Conductive member
- 28p:: Top portion of conductive member located upward of sensor element
- 50:: Wiring board
- C1:: Installation space
- D1:: Downward direction
- F:: Flow path
- G:: Measurement gas
- h1:: Height from sensor element to distal end of protrusion
- h2:: Height from sensor element to inlet port
- h3:: Height from sensor element to outlet port

## Claims

1. A gas sensor, comprising:
a wiring board extending in a longitudinal direction;
a sensor element configured to detect a specific gas component in a measurement gas, the sensor element being disposed inside an outer circumference of one surface of the wiring board and being electrically connected to the wiring board by a plurality of conductive members;
a casing defining an installation space in which the sensor element is installed, the casing having formed thereon an inlet port through which the measurement gas is introduced into the installation space and an outlet port through which the measurement gas is discharged out from the installation space; and
a pretreatment unit configured to pretreat the measurement gas so as to adjust the concentration of the specific gas component in the measurement gas, and then, feed the pretreated measurement gas to the inlet port,
wherein, assuming that a direction in which the sensor element faces the wiring board is a downward direction, the inlet and outlet ports are located at positions outside an outer circumference of the sensor element and upward of the sensor element,
wherein the gas sensor comprises a protrusion provided at a position midway in a flow path of the installation space from the inlet port to the outlet port and facing the sensor element such that the protrusion protrudes toward the inside of the casing so as to narrow the flow path,
wherein a height from the sensor element to a distal end of the protrusion in a direction perpendicular to the longitudinal direction of the wiring board is lower than heights from the sensor element to the inlet and outlet ports in the direction perpendicular to the longitudinal direction of the wiring board, and
wherein the protrusion is disposed more inside than respective top portions of the plurality of conductive members, which are located upward of the sensor element, without being in contact with the conductive members.

2. The gas sensor according to claim 1,
wherein the casing is formed from a metal plate, and
wherein the protrusion is formed integral with the casing.
